# EUROPÄISCHE PATENTSCHRIFT

(11) **EP 3 277 227 B1**
(45) Veröffentlichungstag und Bekanntmachung des Hinweises auf die Patenterteilung: **25.11.2020**
(21) Anmeldenummer: 16712930.3
(22) Anmeldetag: 01.04.2016
(51) Int. Cl.: A61F 2/38, A61F 2/30

(54) **GELENKIMPLANTATTEIL, GELENKENDOPROTHESE UND VERFAHREN ZUM HERSTELLEN EINES GELENKIMPLANTATTEILS UND EINER GELENKENDOPROTHESE**
JOINT IMPLANT PART, JOINT ENDOPROSTHESIS, AND METHOD FOR PRODUCING A JOINT IMPLANT PART AND A JOINT ENDOPROSTHESIS
ÉLÉMENT D'IMPLANT D'ARTICULATION, ENDOPROTHÈSE D'ARTICULATION ET PROCÉDÉ DE FABRICATION D'UN ÉLÉMENT D'IMPLANT D'ARTICULATION ET D'UN ENDOPROTHÈSE D'ARTICULATION

(30) Priorität: 01.04.2015 DE 102015105100
(43) Veröffentlichungstag der Anmeldung: 07.02.2018
(73) Patentinhaber: Aesculap AG, 78532 Tuttlingen (DE)
(72) Erfinder: HAGEN, Thomas, 78532 Tuttlingen (DE); GRUPP, Thomas, 78588 Denkingen (DE)
(74) Vertreter: Hoeger, Stellrecht & Partner Patentanwälte mbB
(86) Internationale Anmeldenummer: PCT/EP2016/057193
(87) Internationale Veröffentlichungsnummer: WO 2016/156561

(56) Entgegenhaltungen:
- EP-A1- 2 676 636
- WO-A1-2013/109422
- WO-A2-2007/053022
- GB-A- 2 045 082
- US-A1- 2007 116 734
- US-A1- 2011 202 140

## Beschreibung

Die vorliegende Erfindung betrifft ein Gelenkimplantatteil einer Gelenkendoprothese.

Ferner betrifft die vorliegende Erfindung eine Gelenkendoprothese umfassend mindestens ein Gelenkimplantatteil.

Des Weiteren betrifft die vorliegende Erfindung ein Verfahren zum Herstellen eines Gelenkimplantatteils einer Gelenkendoprothese.

Außerdem betrifft die vorliegende Erfindung ein Verfahren zum Herstellen einer Gelenkendoprothese umfassend mindestens ein Gelenkimplantatteil.

Gelenkimplantatteile, welche mindestens einen ersten Volumenbereich, mindestens einen zweiten Volumenbereich und mindestens einen dritten Volumenbereich umfassen, welcher mindestens eine erste Volumenbereich einen Knochenanlageflächenbereich mit mindestens einer Knochenanlagefläche definiert, welcher mindestens eine zweite Volumenbereich einen Gelenkflächenbereich mit mindestens einer Gelenkfläche definiert und welcher mindestens eine dritte Volumenbereich weder eine Knochenanlagefläche noch eine Gelenkfläche umfasst, wobei sich ein Elastizitätsmodul im mindestens einen ersten und/oder im mindestens einen zweiten und/oder im mindestens einen dritten Volumenbereich in mindestens einer Raumrichtung stetig oder im Wesentlichen stetig oder unstetig ändert und wobei sich eine Festigkeit im mindestens einen ersten und/oder im mindestens einen zweiten und/oder im mindestens einen dritten Volumenbereich in mindestens einer Raumrichtung stetig oder im Wesentlichen stetig oder unstetig ändert, und Gelenkendoprothesen der eingangs beschriebenen Art sind in vielfältigen Ausführungsformen bekannt. Sie werden aus zugelassenen Implantatwerkstoffen hergestellt.

Ein Problem bekannter Gelenkendoprothesen ist es, dass es zwischen Knochenanlageflächen der Gelenkimplantatteile und dem zur Anlage eines Gelenkimplantatteils präparierten Knochen zu Mikrobewegungen kommen kann. Der Grund hierfür sind insbesondere Eigenschaften des Materials, aus welchem das Gelenkimplantatteil ausgebildet ist. Diese unterscheiden sich in der Regel signifikant von Eigenschaften des präparierten Knochens. Mikrobewegungen beeinflussen jedoch die Haltbarkeit der Verbindung zwischen dem Gelenkimplantatteil und dem präparierten Knochen negativ.

Aus der WO 2013/109422 A1 ist ein körperverträgliches, nicht-resorbierbares Implantat bekannt. Die US 2011/0202140A1 betrifft tragende Implantate mit gestalteter, sich ändernder Steifigkeit. Künstliche Hüftschäfte sind in der EP 2 676 636 A1 und der GB 2 045 082 A offenbart. Aus der US 2007/0116734 A1 sind poröse, tragende keramische oder metallische Implantate bekannt. Ferner sind in der WO 2007/053022 A2 Implantate und Verfahren zum Herstellen solcher Implantate beschrieben.

Es ist daher eine Aufgabe der vorliegenden Erfindung, ein Gelenkimplantatteil, eine Gelenkendoprothese und Verfahren zum Herstellen eines Gelenkimplantatteils und einer Gelenkendoprothese der eingangs beschriebenen Art so zu verbessern, dass insbesondere eine Lockerungsgefahr zwischen Implantat und Knochen verringert wird.

Diese Aufgabe wird erfindungsgemäß gelöst durch eine Gelenkimplantatteil gemäß Anspruch 1.

Weitere bevorzugte Ausführungsformen werden durch die Unteransprüche definiert.

Die erfindungsgemäß vorgeschlagene Lösung ermöglicht es insbesondere, Gelenkimplantatteile derart optimiert auszubilden, dass im Übergang vom Implantatteil zum Knochen, also im Knochenanlageflächenbereich und insbesondere an der Knochenanlagefläche, eine Elastizität des Gelenkimplantatteils an eine Elastizität des präparierten Knochens angepasst ist. Auf diese Weise können Mikrobewegungen verhindert werden, die bei herkömmlichen Gelenkimplantatteilen, die aus einem Werkstoff mit einem gleichmäßig verteilten Gefüge mit einer entsprechenden Elastizität hergestellt werden, auftreten können. Insbesondere ermöglicht es die vorgeschlagene Weiterbildung, Elastizitäten der unterschiedlichen Volumenbereiche des Gelenkimplantatteils unterschiedlich einzustellen und zudem auch in den jeweiligen Volumenbereichen in der angegebenen Weise zu ändern, um sowohl innerhalb des Gelenkimplantatteils als auch im Übergang zu einem Knochen oder einem anderen Gelenkimplantatteil beispielsweise stetige Änderungen des Elastizitätsmoduls vorzugeben. Auf diese Weise kann das Gelenkimplantatteil optimal an den Knochen und ein zur Ausbildung der Gelenkendoprothese weiteres Gelenkimplantatteil angepasst werden, um insgesamt insbesondere eine Struktur und/oder eine Funktion des Gelenkimplantats an das ursprüngliche Gelenk anzupassen. So kann eine Dauerstabilität der Gelenkendoprothese im Vergleich zu herkömmlichen bekannten Endoprothesen verbessert werden. Ferner ist es günstig, dass sich eine Festigkeit im mindestens einen ersten und/oder im mindestens einen zweiten und/oder im mindestens einer Raumrichtung stetig oder im Wesentlichen stetig oder unstetig ändert. Die Festigkeit in der angegebenen Weise in mindestens einem der drei verschiedenen Volumenbereiche zu ändern, gestattet es insbesondere, Bereiche des Gelenkimplantatteils entsprechend den Festigkeitsanforderungen mit einer höheren oder geringeren Festigkeit auszubilden. Dabei können Festigkeitsänderungen stetig oder unstetig erfolgen. Insbesondere können Bereiche geringerer Festigkeit beispielsweise weniger massiv ausgeführt werden als dies bei herkömmlichen Gelenkimplantatteilen der Fall ist, die in der Regel aus einem Werkstoff mit einem gleichmäßig verteilten Gefüge gefertigt sind. Auf diese Weise lassen sich Gelenkimplantatteile ausbilden, die in ihrer Struktur einem natürlichen Gelenkteil, welches sie ersetzen sollen, möglichst nahe kommen. Um das Gelenkimplantatteil möglichst leicht auszubilden, ist es vorteilhaft, dass der mindestens erste Volumenbereich und der mindestens eine zweite Volumenbereich und der mindestens eine dritte Volumenbereich mindestens einen Hohlraum aufweisen. Es können insbesondere mehrere Hohlräume vorgesehen sein oder auch eine Mehrzahl von Hohlräumen, die miteinander verbunden oder voneinander getrennt sind. Sind mehrere Hohlräume vorgesehen, können diese im Wesentlichen gleich groß ausgebildet sein oder auch deutlich unterschiedliche Größen und Formen aufweisen. Günstig ist es, dass eine Mehrzahl von Hohlräumen ausgebildet ist und dass ein durch die Mehrzahl von Hohlräumen definiertes Gesamtvolumen im mindestens einen dritten Volumenbereich größer ist als im mindestens einen ersten und im mindestens einen zweiten Volumenbereich. Der mindestens eine dritte Volumenbereich, welcher weder eine Knochenanlagefläche noch eine Gelenkfläche definiert, kann so mit einem maximal großen Hohlraumvolumen ausgebildet werden, beispielsweise um das Einwachsen von Knochen zu erleichtern, wenn die Mehrzahl von Hohlräumen miteinander in Fluidverbindung stehen, oder um ein Gewicht des Gelenkimplantatteils zu minimieren, insbesondere dadurch, dass dort, wo nur geringe Festigkeiten erforderlich sind, die Zahl und/oder das Volumen der Hohlräume maximiert wird. Um die Implantation zu vereinfachen und auch eine Stabilität des Gelenkimplantatteils zu verbessern, ist es vorteilhaft, dass das Gelenkimplantatteil einstückig ausgebildet ist. Da keine weiteren Verbindungen erforderlich sind, kann so auch ein Gewicht des Gelenkimplantatteils minimiert werden. Gemäß der Erfindung ist vorgesehen, dass das Gelenkimplantatteil in Form eines an einem Femur festlegbaren Femurteils einer Kniegelenkendoprothese ausgebildet ist. Prinzipiell lassen sich Gelenkimplantatteile beliebiger Gelenke des menschlichen Körpers in der erfindungsgemäß vorgeschlagenen Weise ausbilden, auch die noch nicht erwähnten Zwischenwirbelimplantate.

Vorzugsweise weist der mindestens eine erste Volumenbereich eine Elastizität mit einem Elastizitätsmodul auf, insbesondere im Bereich der mindestens einen Knochenanlagefläche, welcher einem Elastizitätsmodul eines menschlichen oder tierischen Knochens entspricht oder im Wesentlichen entspricht. Entspricht insbesondere der Elastizitätsmodul im Knochenanlageflächenbereich sowie im Bereich der Knochenanlagefläche einem Elastizitätsmodul eines menschlichen oder tierischen Knochens oder entspricht diesem im Wesentlichen oder kommt diesem nahe, an welchen das Gelenkimplantatteil mit der Knochenanlagefläche festgelegt werden soll, dann ergibt sich ein stetiger oder im Wesentlichen stetiger Übergang des Elastizitätsmoduls des Gelenkimplantatteils einerseits und des Knochens andererseits. Dadurch lassen sich insbesondere Mikrobewegungen zwischen dem Gelenkimplantatteil und dem Knochen minimieren oder gar ganz vermeiden.

Vorzugsweise liegt ein Elastizitätsmodul des mindestens einen ersten und/oder des mindestens einen zweiten und/oder des mindestens einen dritten Volumenbereichs in einem Bereich von etwa 0,2 kN/mm² bis etwa 100 kN/mm². Vorzugsweise liegt der Elastizitätsmodul in einem Bereich von etwa 0,2 kN/mm² bis etwa 20 kN/mm². Ein Elastizitätsmodul in des angegebenen Bereichen für mindestens einen der mindestens drei Volumenbereiche vorzusehen, ermöglicht insbesondere eine Anpassung der Elastizität des Gelenkimplantatteils an einem natürlichen Knochen auf einfache Weise.

Vorteilhaft ist es, wenn die Festigkeit des mindestens einen ersten und/oder des mindestens einen zweiten und/oder des mindestens einen dritten Volumenbereichs in einem Bereich von etwa 0,2 kN/mm² bis etwa 100 kN/mm² liegt. Vorzugsweise liegt die Festigkeit in einem Bereich von etwa 0,2 kN/mm² bis etwa 20 kN/mm². Festigkeiten in den angegebenen Bereichen vorzusehen ermöglicht es insbesondere, Implantatteile in den Bereichen, in denen eine erhöhte Festigkeit erforderlich ist, hinreichend stabil auszubilden, jedoch in Bereichen, die nur eine geringe Festigkeit erfordern, eine Festigkeit zu minimieren, beispielsweise um Material und damit Gewicht einzusparen. So kann insbesondere bei Revisionsimplantaten, beispielsweise im Kniebereich, ein herkömmliches Gelenkimplantatteil mehrere Kilogramm wiegen, was von Patienten als unangenehm empfunden wird. Gelenkimplantatteile lassen sich durch die vorgeschlagene Weiterbildung deutlich leichter ausbilden, da beispielsweise durch FEM-Berechnungen Festigkeiten in Abhängigkeit der Implantatform vorgegeben und optimiert werden können.

Eine Anpassung des Gelenkimplantatteils an die Bedürfnisse eines Patienten lässt sich weiter verbessern, wenn der mindestens eine erste Volumenbereich eine innere Struktur aufweist, welche im Wesentlichen einem inneren Aufbau eines menschlichen oder tierischen Knochens entspricht. Beispielsweise können Knochenbälkchen in künstlichen Gelenkimplantatteilen nachgebildet werden. Insbesondere ist dies eine Möglichkeit, eine Elastizität und/oder eine Festigkeit des Knochenanlagebereichs und der Knochenanlagefläche an Eigenschaften eines natürlichen Knochens anzupassen. Mikrobewegungen werden so minimiert, sodass Lockerungen des implantierten Gelenkimplantatteils im Wesentlichen vermieden werden.

Auf besonders einfache Weise lässt sich das Gelenkimplantatteil herstellen, wenn es durch Sintern aus mindestens einem sinterfähigen Material hergestellt ist. Insbesondere kann es durch Lasersintern hergestellt sein. Das Gelenkimplantatteil kann jedoch auch aus mehreren Materialien gesintert werden, die beispielsweise zur Ausbildung unterschiedlicher Volumenbereiche eingesetzt werden.

Günstig ist es, wenn das Gelenkimplantatteil aus mindestens zwei Materialien hergestellt ist. Insbesondere können diese vor dem Sintern, beispielsweise dem Lasersintern, miteinander gemischt werden. Auf diese Weise lassen sich Eigenschaften eines natürlichen Knochens noch besser nachbilden.

Vorzugsweise ist das mindestens eine sinterfähige Material ein Metall und/oder einen Kunststoff. So können beispielsweise Metalle und Kunststoffe miteinander gemischt und zu einem Gelenkimplantat in einem einzigen Verfahrensschritt beispielsweise durch Sintern ausgebildet werden.

Vorzugsweise ist das Metall Kobalt, Chrom und/oder Molybdän oder enthält eins oder mehrere der genannten Metalle. So lassen sich beispielsweise Gelenkimplantate aus Metallen ausbilden, die bereits als Implantatwerkstoffe zugelassen sind.

Günstig ist es, wenn der Kunststoff Polyetheretherketon ist oder enthält. Polyetheretherketon (PEEK) ist als Implantatwerkstoff hervorragend geeignet und ermöglicht es insbesondere, Elastizitäten und Festigkeiten bei einem Gelenkimplantatteil einzustellen, die denen eines natürlichen Knochens entsprechen.

Um insbesondere Elastizitäten und/oder Festigkeiten unterschiedlicher Volumenbereiche des Gelenkimplantatteils möglichst individuell einstellen zu können, ist es günstig, wenn der mindestens eine erste und/oder der mindestens eine zweite und/oder der mindestens eine dritte Volumenbereich aus unterschiedlichen Materialien ausgebildet sind. Beispielsweise kann der erste Volumenbereich aus einem Metall ausgebildet sein, der zweite aus einer Keramik und der dritte aus einer Mischung aus einem Metall und einem Kunststoff.

Gemäß einer weiteren bevorzugten Ausführungsform der Erfindung kann vorgesehen sein, dass sich ein Anteil unterschiedlicher Materialen beim Übergang zwischen zwei der drei unterschiedlichen Volumenbereiche in mindestens einer Raumrichtung stetig oder im Wesentlichen stetig ändert oder unstetig ändert. Insbesondere bei einer stetigen oder im Wesentlichen stetigen Änderung eines Anteils der unterschiedlichen Materialien beim Übergang zwischen zwei der drei Volumenbereiche kann insgesamt ein homogenes Gelenkimplantatteil ausgebildet werden. Unter homogen ist hier insbesondere nicht zu verstehen, dass das gesamte, vom Gelenkimplantatteil definierte Volumen ein einheitliches Gefüge aufweist.

Insbesondere ist es günstig wenn der mindestens eine Hohlraum in Form eines abgeschlossenen, vollständig umschlossenen Hohlraums ausgebildet ist. Auf diese Weise kann verhindert werden, dass Knochenmaterial in den Hohlraum hineinwächst oder Materialien, die vor der Ausbildung des Gelenkimplantatteils oder beim Ausbilden desselben in den Hohlraum eingebracht wurden, nach außen und in den Körper eines Patienten gelangen können.

Um eine Verbindung zwischen dem Gelenkimplantatteil und einem Knochen zu verbessern, ist es vorteilhaft, wenn eine Mehrzahl von Hohlräumen ausgebildet ist und wenn mindestens ein Teil der Hohlräume miteinander in Fluidverbindung steht. Ferner ermöglichen in Fluidverbindung stehende Hohlräume insbesondere auch eine optimierte Reinigung des Gelenkimplantatteils nach dessen Herstellung. Zudem können gezielt auch andere Materialien und gegebenenfalls auch Medikamente in Hohlräume des Gelenkimplantatteils eingebracht werden.

Um eine Verbindung des Gelenkimplantatteils im Bereich des Knochenanlageflächenbereichs mit dem präparierten Knochen des Patienten zu verbessern, ist es vorteilhaft, wenn der mindestens eine Hohlraum in Form einer Vertiefung der Knochenanlagefläche ausgebildet ist.

Vorzugsweise ist die mindestens eine Gelenkfläche in Form einer geschlossenen Materialschicht und/oder glatt und/oder poliert ausgebildet. Dies ermöglicht es insbesondere, Gelenkimplantatteile mit optimierten Gelenkflächen auszubilden. Beispielsweise kann ein Volumenbereich des Gelenkimplantatteils, welches keine Gelenkfläche definiert, aus einem anderen Material ausgebildet werden als ein zweiter Volumenbereich des Gelenkimplantatteils. Beispielsweise kann der zweite Volumenbereich aus einer Keramik gebildet sein, der dritte Volumenbereich aus einem Metall. Ein Übergang zwischen beiden Volumenbereichen kann stetig, im Wesentlichen stetig oder unstetig erfolgen.

Die eingangs gestellte Aufgabe wird ferner bei einer Gelenkendoprothese der eingangs beschriebenen Art erfindungsgemäß dadurch gelöst, dass das mindestens eine Gelenkimplantatteil in Form eines der oben beschriebenen Gelenkimplantatteile ausgebildet ist. Die Gelenkendoprothese weist dann ebenfalls die oben in Verbindung mit bevorzugten Ausführungsformen von Gelenkimplantatteilen beschriebenen Vorteile auf.

Günstig ist es, wenn die Gelenkendoprothese in Form einer Kniegelenkendoprothese ausgebildet ist. So lassen sich Gelenke des menschlichen oder tierischen Körpers durch Gelenkendoprothesen ersetzen, die optimal an Eigenschaften des Knochens, mit dem sie verbunden werden, angepasst sind.

Die eingangs gestellte Aufgabe wird ferner erfindungsgemäß durch ein Verfahren gemäß Anspruch 10 gelöst.

Das erfindungsgemäß vorgeschlagene Verfahren ermöglicht es insbesondere, ein Gelenkimplantatteil so auszubilden, dass es beispielsweise eine Elastizität im Bereich einer Knochenanlagefläche aufweist, die einer Elastizität des natürlichen Knochens entspricht. Wie bereits oben eingehend dargelegt, können so Mikrobewegungen zwischen dem Gelenkimplantatteil und dem Knochen nach der Implantation vermieden oder zumindest signifikant verringert werden. Im Vergleich zu herkömmlichen Gelenkimplantatteilen kann so die Gefahr von Lockerungen des Gelenkimplantatteils aufgrund der Mikrobewegungen minimiert oder sogar ganz ausgeschlossen werden. Vorteilhaft ist es ferner, dass der mindestens eine erste und/oder der mindestens eine zweite und/oder der mindestens eine dritte Volumenbereich mit einer Festigkeit ausgebildet werden, welche sich in mindestens einer Raumrichtung stetig oder im Wesentlichen stetig oder unstetig ändert. Das bekannte Verfahren in der vorgeschlagenen Weise weiterzubilden ermöglicht es insbesondere, das Gelenkimplantatteil mit unterschiedlichen Festigkeiten auszubilden. Insbesondere können die mindestens drei definierten Volumenbereiche unterschiedliche Festigkeit aufweisen. So lassen sich Gelenkimplantatteile optimieren, insbesondere hinsichtlich ihres Gewichts. Ferner ist es vorteilhaft, dass der mindestens eine erste Volumenbereich und der mindestens eine zweite Volumenbereich und der mindestens eine dritte Volumenbereich mit mindestens einem Hohlraum ausgebildet werden. Zum einen kann so Material gespart werden, was hilft, das Gewicht des Gelenkimplantatteils zu reduzieren, zum anderen können so auch Hohlräume genutzt werden, in die Knochen- oder Körpergewebe einwachsen kann, um eine Verbindung des Gelenkimplantatteils mit einem Knochen zu verbessern. Vorteilhaft ist es, dass eine Mehrzahl von Hohlräumen ausgebildet wird derart, dass ein durch die Mehrzahl von Hohlräumen definiertes Gesamtvolumen im mindestens einen dritten Volumenbereich größer ist als im mindestens einen ersten und/oder im mindestens einen zweiten Volumenbereich. Insbesondere der mindestens eine dritte Volumenbereich kann maximal hohl ausgebildet werden. Dies hilft, ein Gewicht des Gelenkimplantatteils zu reduzieren. Vorzugsweise sind dabei diejenigen Bereiche des Gelenkimplantatteils mit vielen Hohlräumen mit großem Gesamtvolumen versehen, für die nur geringe Anforderungen an die Festigkeit eingehalten werden müssen. Um geschädigte natürliche Gelenke von Menschen oder Tieren ersetzen zu können, ist es vorteilhaft, dass das Gelenkimplantatteil in Form eines an einem Femur festlegbaren Femurteils einer Kniegelenkendoprothese ausgebildet wird. Gemäß der Erfindung wird das Gelenkimplantatteil einstückig ausgebildet. Es kann dann in einem Zug hergestellt werden, wodurch Herstellungskosten gesenkt werden können.

Um eine optimale Anpassung des Gelenkimplantats an einen Knochen zu ermöglichen, ist es vorteilhaft, wenn der mindestens eine erste Volumenbereich mit einer Elastizität mit einem Elastizitätsmodul ausgebildet wird, insbesondere im Bereich der mindestens einen Knochenanlagefläche, welcher einem Elastizitätsmodul eines menschlichen oder tierischen Knochens entspricht.

Um eine möglichst optimale Anpassung des Gelenkimplantatteils an einen menschlichen oder tierischen Knochen zu ermöglichen, ist es günstig, wenn der mindestens eine erste und/oder der mindestens eine zweite und/oder der mindestens eine dritte Volumenbereich mit einem Elastizitätsmodul ausgebildet werden, welcher in einem Bereich von etwa 0,2 kN/mm² bis etwa 100 kN/mm² liegt. Vorzugsweise liegt der Elastizitätsmodul in einem Bereich von etwa 0,2 kN/mm² bis etwa 20 kN/mm².

Um eine hinreichende Festigkeit des Gelenkimplantatteils sicherstellen zu können, ist es vorteilhaft, wenn der mindestens eine erste und/oder der mindestens eine zweite und/oder der mindestens eine dritte Volumenbereich mit einer Festigkeit ausgebildet werden, welche in einem Bereich von etwa 0,2 N/m² bis etwa 100 N/m² liegt. Vorzugsweise liegt die Festigkeit in einem Bereich von etwa 0,2 kN/mm² bis etwa 20 kN/mm².

Des Weiteren kann es günstig sein, wenn der mindestens eine erste Volumenbereich mit einer inneren Struktur ausgebildet wird, welche im Wesentlichen einem inneren Aufbau eines menschlichen oder tierischen Knochens entspricht. Insbesondere können Knochenbälkchen des natürlichen Knochens durch geeignete Materialien, beispielsweise Metalle oder Kunststoffe, nachgebildet werden.

Günstigerweise wird das Gelenkimplantatteil durch Sintern, aus mindestens einem sinterfähigen Material hergestellt. Insbesondere kann das Gelenkimplantatteil durch Lasersintern hergestellt werden. Beispielsweise kann das Sintern auch im Rahmen eines sogenannten 3D-Drucks erfolgen, um das Gelenkimplantatteil im Rahmen eines additiven Fertigungsverfahrens herzustellen. Selbstverständlich können auch zwei oder mehr unterschiedliche sinterfähige Materialien eingesetzt werden, um das Gelenkimplantatteil auszubilden.

Günstig ist es, wenn das Gelenkimplantatteil aus mindestens zwei Materialien hergestellt wird, welche vor dem Sintern gemischt werden. Die Mischung kann homogen oder inhomogen sein, je nachdem ob ein homogener Aufbau des Gelenkimplantatteils gewünscht wird oder nicht.

Vorzugsweise werden als das mindestens eine sinterfähige Material mindestens ein Metall und/oder mindestens einen Kunststoffe eingesetzt. Insbesondere kann es sich dabei um körperverträgliche Materialien handeln.

Das Verfahren lässt sich auf einfache Weise durchführen, wenn als das mindestens eine Metall Titan, Kobalt, Chrom und/oder Molybdän oder eine Mischung aus zwei oder mehreren dieser Metalle oder Legierungen dieser Metalle eingesetzt werden. Diese Metalle eignen sich hervorragend als Implantatwerkstoffe und weisen die erforderliche Körperverträglichkeit auf.

Einfach und kostengünstig durchführen lässt sich das Verfahren zudem, wenn als der mindestens eine Kunststoff Polyetheretherketon verwendet wird. Polyetheretherketon (PEEK) eignet sich hervorragend als Implantatwerkstoff und lässt sich sehr gut verarbeiten, insbesondere auch in einem Gemisch mit einem Metallpulver.

Vorteilhaft ist es, wenn der mindestens eine erste und/oder der mindestens eine zweite Volumenbereich und/oder der mindestens eine dritte Volumenbereich aus unterschiedlichen Materialien ausgebildet werden. Auf diese Weise können Gelenkimplantatteile optimiert gestaltet werden, insbesondere im Bereich einer Gelenkfläche sowie auch im Bereich einer Knochenanlagefläche. Zudem kann ein Gewicht des Gelenkimplantatteils minimiert werden, wenn insbesondere im mindestens einen dritten Volumenbereich sehr leichte Materialien eingesetzt werden.

Gemäß einer weiteren bevorzugten Variante des Verfahrens kann vorgesehen sein, dass das Gelenkimplantatteil derart ausgebildet wird, dass sich ein Anteil unterschiedlicher Materialien beim Übergang zwischen zwei der drei unterschiedlichen Volumenbereiche in mindestens einer Raumrichtung stetig oder im Wesentlichen stetig ändert oder unstetig ändert. Durch diese Verfahrensvariante wird es insbesondere ermöglicht, besonders stabile Gelenkimplantatteile auszubilden, da nicht zwingend unstetige Änderungen bei den Materialien erforderlich sind. Vielmehr können unterschiedliche Materialien, ähnlich wie ineinander verwachsenes Gewebe, einander durchsetzen und so eine Stabilität des Gelenkimplantatteils optimieren.

Günstig ist es, wenn der mindestens eine Hohlraum in Form eines abgeschlossenen, vollständig umschlossenen Hohlraums ausgebildet wird. Auf diese Weise kann insbesondere verhindert werden, dass beispielsweise in den Hohlraum einbrachte Füllstoffe austreten können. Dies ist insbesondere dann von Bedeutung, wenn die Füllstoffe nicht körperverträgliche Füllstoffe sind.

Günstig ist es, wenn eine Mehrzahl von Hohlräumen ausgebildet wird derart, dass mindestens ein Teil der Hohlräume miteinander in Fluidverbindung steht. Das Verfahren so durchzuführen ermöglicht es insbesondere, Knochen oder Gewebe in die miteinander in Fluidverbindung stehenden Hohlräume einwachsen zu lassen.

Besonders einfach durchführen lässt sich das Verfahren, wenn der mindestens eine Hohlraum in Form einer Vertiefung der Knochenanlagefläche ausgebildet wird. Beispielsweise kann diese im Rahmen des Sinterns oder nachträglich durch Bohren, Fräsen oder eine beliebige andere spanende Bearbeitung ausgebildet werden.

Eine besonders langzeitstabile Gelenkendoprothese lässt sich erreichen, wenn die mindestens eine Gelenkfläche in Form einer geschlossenen Materialschicht und/oder glatt und/oder poliert ausgebildet wird. Auf diese Weise kann eine Reibung zwischen dem Gelenkimplantatteil und einem mit diesem zusammenwirkenden weiteren Gelenkimplantatteil zur Ausbildung der Gelenkendoprothese minimiert werden.

Ferner wird die eingangs gestellte Aufgabe bei einem Verfahren zum Herstellen einer Gelenkendoprothese der eingangs beschriebenen Art erfindungsgemäß dadurch gelöst, dass das mindestens eine Gelenkimplantatteil durch eines der oben beschriebenen Verfahren ausgebildet wird. Das Verfahren zum Herstellen der Gelenkendoprothese weist dann ebenfalls die oben beschriebenen Vorteile auf.

Vorzugsweise wird die Gelenkendoprothese in Form einer Kniegelenkendoprothese ausgebildet. So können Gelenke, welche in Folge eines Traumas oder einer Abnutzung beschädigt sind, durch eine künstliche Gelenkendoprothese ersetzt werden.

Die nachfolgende Beschreibung bevorzugter Ausführungsformen der Erfindung dient im Zusammenhang mit der Zeichnung der näheren Erläuterung. Es zeigen:
- Figur 1:: eine schematische Darstellung zweier möglicher Schnitte durch ein dreidimensionales Gelenkimplantatteil;
- Figur 2:: eine schematische perspektivische Ansicht eines Gelenkimplantatteils;
- Figur 3:: eine schematische Darstellung unterschiedlicher Elastizitätsverläufe eines Gelenkimplantatteils in Abhängigkeit einer Implantatdicke;
- Figur 4:: eine schematische Schnittansicht durch ein Gelenkimplantatteil; und
- Figur 5:: eine Ausschnittsansicht im Übergangsbereich zwischen einem Gelenkimplantat und einem Knochen.

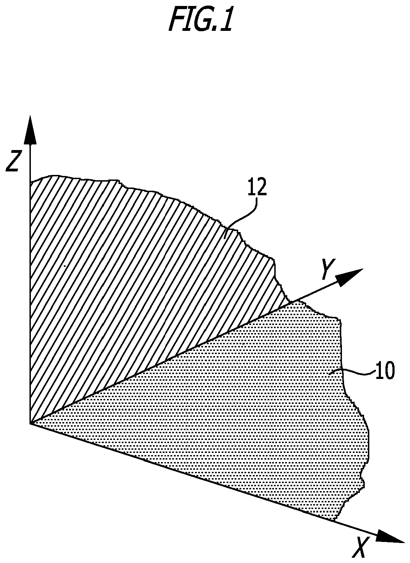

Um die Darstellbarkeit einer neuen dreidimensionalen Bauteilgeometrie zu vereinfachen, sind in Figur 1 die räumlichen Verhältnisse in jeweils zwei flächigen Geometrien dargestellt. Dies sind zum einen die xy-Ebene 10 sowie die yz-Ebene 12. Die xy-Ebene 10 symbolisiert dabei beispielsweise die Sicht auf eine Implantatoberfläche, die yz-Ebene 12 einen Querschnitt durch eine Implantatdicke.

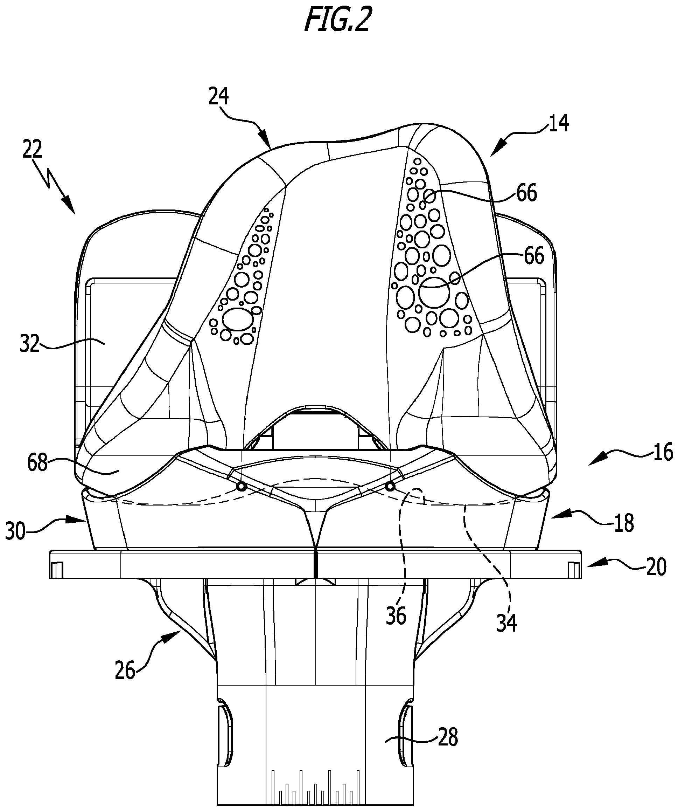

In Figur 2 ist schematisch ein Gelenkimplantatteil 14 einer insgesamt mit dem Bezugszeichen 16 bezeichneten Gelenkendoprothese dargestellt. Die Gelenkendoprothese 16 umfasst ferner zwei weitere Gelenkimplantatteile 18 und 20. Die Gelenkendoprothese 10 in Figur 2 schematisch als eine Kniegelenkendoprothese 22 dargestellt mit einem Femurteil 24 zum Verankern an einem Femurknochen, einem Tibiateil 26 mit einem Schaft 28 zum Verankern an einem Tibiaknochen und einem zwischen dem Tibiateil 26 und dem Femurteil 24 angeordneten Meniskusteil 30.

Am Femurteil 24 ist eine Knochenanlagefläche 32 ausgebildet sowie eine Gelenkfläche 34, welche mit einer korrespondierenden Gelenkfläche 36 des Gelenkimplantatteils 18 nach der Implantation zusammenwirkt.

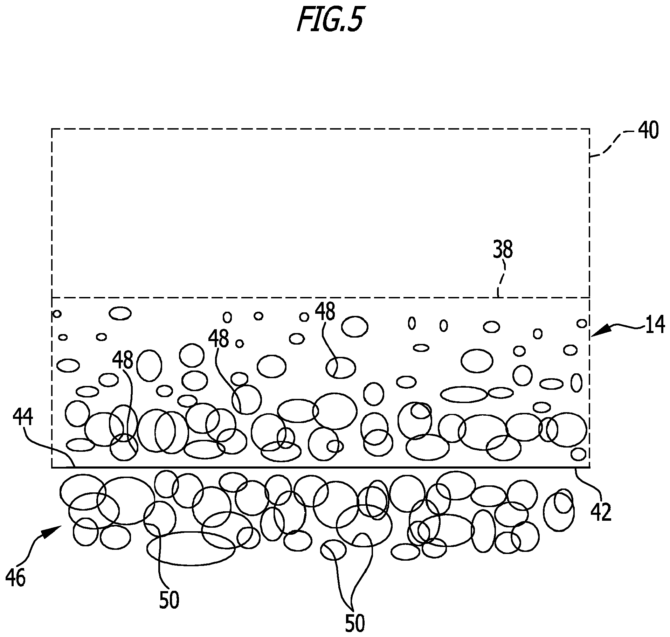

In der in Figur 1 dargestellten xy-Ebene geht es in erster Linie um äußere Flächen des Gelenkimplantatteils 14, also insbesondere um die Knochenanlagefläche 32 sowie die Gelenkfläche 34. Das Augenmerk liegt hierbei auf einer sinnvollen Verteilung der Elastizität im Bereich eines Kontakts mit einem Knochen. Wie in Figur 5 schematisch dargestellt, wird dabei ein Knochenanlagebereich 38 definiert und gegenüber einem inneren Bereich 40 des Gelenkimplantatteils 14 abgegrenzt. Der Knochenanlageflächenbereich 38 definiert eine Knochenanlagefläche 42, welche flächig an einer präparierten Knochenfläche 44 eines in Figur 5 ausschnittsweise dargestellten Knochens 46 anliegt.

Eine Struktur des Knochenanlageflächenbereichs 38 ist an eine Struktur des präparierten Knochens 46 angepasst. Wie in Figur 5 gut zu erkennen, ist im Knochenanlageflächenbereich 42 eine Mehrzahl von Hohlräumen 48 unterschiedlicher Größe ausgebildet, sodass eine poröse Struktur gebildet wird, welche einer Struktur des Knochens 46, welcher ebenfalls eine Mehrzahl von Hohlräumen unterschiedlicher Größe aufweist, entspricht. Durch diese Ausgestaltung des Knochenanlageflächenbereichs 38 wird eine Elastizität dieses Bereichs des Gelenkimplantatteils 14 so gestaltet und vorgegeben, dass die Elastizität in etwa der Elastizität des präparierten Knochens 46 entspricht. Dies kann also wie beschrieben zum einen insbesondere über die innere Struktur des Knochenanlageflächenbereichs 38 erreicht werden, zum anderen über das oder die zur Herstellung des Gelenkimplantatteils 14 verwendeten Materialien.

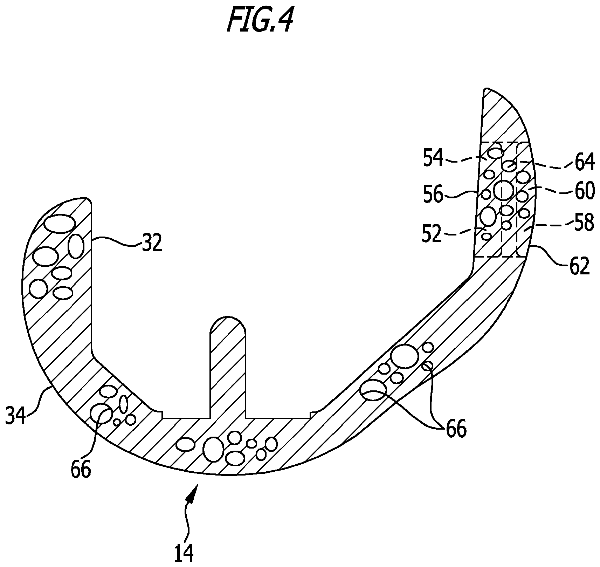

Die in Figur 1 bezeichnete yz-Ebene 12 symbolisiert wie beschrieben einen Schnitt durch das Implantat. Eine schematische Schnittansicht beispielsweise des Gelenkimplantatteils 14 ist in Figur 4 dargestellt.

In Figur 4 sind auch schematisch und exemplarisch drei unterschiedliche Volumenbereiche dargestellt, nämlich ein erster Volumenbereich 52, welcher einen Knochenanlageflächenbereich 54 mit einer Knochenanlagefläche 56 definiert, ein zweiter Volumenbereich 58, welcher einen Gelenkflächenbereich 60 mit einer Gelenkfläche 62 definiert und einen dritten Volumenbereich 64. Dieser weist weder eine Knochenanlagefläche noch eine Gelenkfläche auf, befindet sich also vollständig im Inneren des Gelenkimplantatteils 14.

Die in Figur 4 schematisch eingezeichneten Volumenbereiche 52, 58 und 64 bilden somit jeweils einen Teil des Gelenkimplantatteils 14. Es können von jeder Art der drei Volumenbereiche weitere Volumenbereiche in einem Gelenkimplantatteil definiert werden, die die angegebenen Eigenschaften aufweisen.

Ein wesentliches Ziel einer Gestaltung in der yz-Ebene ist der Leichtbau des Gelenkimplantatteils 14. Mit Hilfe theoretischer Überlegungen, beispielsweise mittels Berechnungen unter Einsatz von Finite-Elemente-Verfahren, können unterschiedlich belastete Bereiche am Gelenkimplantatteil 14 lokalisiert werden. Um eine ausreichende Stabilität des Gelenkimplantatteils 14 gewährleisten zu können, werden dann hochbelastete Bereiche stabiler ausgelegt als weniger oder nicht belastete Bereiche.

Maßnahmen des Leichtbaus stellen insbesondere das Weglassen von Material in größeren oder kleineren Mengen dar. Dies äußert sich durch die Ausbildung von Hohlräumen 66 in denjenigen Bereichen, die weniger stark belastet sind, also eine geringere Festigkeit aufweisen müssen als hochbelastete Bereiche. Dies kann beispielweise in Figur 4 direkt daran abgelesen werden, wie groß eine Dichte der Hohlräume 66 ist. Beispielsweise können in Randbereichen, also bis hin zur Gelenkfläche 34 oder zur Knochenanlagefläche 32 Hohlräume 66 ausgebildet werden.

Bei der Knochenanlagefläche 32 handelt es sich insbesondere um eine Fläche zur Verankerung beziehungsweise Fixierung des Gelenkimplantatteils 14 am Knochen. Die Verankerung und Fixierung kann auf diese Weise signifikant beeinflusst werden. Eine offenporige Struktur der Knochenanlagefläche 32 ermöglicht das Einwachsen von Knochen in das Gelenkimplantatteil.

Eine weniger feste Struktur des Gelenkimplantatteils 14 kann auch komplett unsichtbar ausgebildet werden, beispielsweise wenn eine glatte, polierte Oberfläche 68 erforderlich ist, welche mit der Gelenkfläche 34 des weiteren Gelenkimplantatteils 18 zusammenwirkt. Insbesondere in Verlängerungsschäften von Knie-Revisions-Endoprothesen ist eine Verringerung des Gewichts von großem Interesse. Auch in geringer belasteten Bereichen eines Hüftschafts einer Hüftgelenkendoprothese ist das Weglassen von Material und somit eine Verringerung des Gewichts des Gelenkimplantatteils von Bedeutung.

Die beschriebenen Gelenkimplantatteile können insbesondere durch Sintern, beispielsweise Lasersintern, aus einem einzigen Material ausgebildet werden. Alternativ können auch ein, zwei oder mehrere weitere Materialien mit einem Grundmaterial vermischt werden. Dies kann beispielsweise ein Metallpulver sein, welches Kobalt, Chrom und/oder Molybdän enthält, welches optional mit einem Kunststoff, beispielsweise Polyetheretherketon gemischt und anschließend mit einem Laser gesintert wird.

In den drei unterschiedlichen Volumenbereichen 52, 58 und 64 können die Anteile der Materialien unterschiedlich sein. Ferner können auch die Hohlräume 66 unterschiedlich groß und in unterschiedlicher Anzahl ausgebildet werden. Auf diese Weise lassen sich Elastizitätsverläufe mit unterschiedlichen Elastizitätsmodulen in Abhängigkeit einer Implantatdicke nahezu beliebig einstellen.

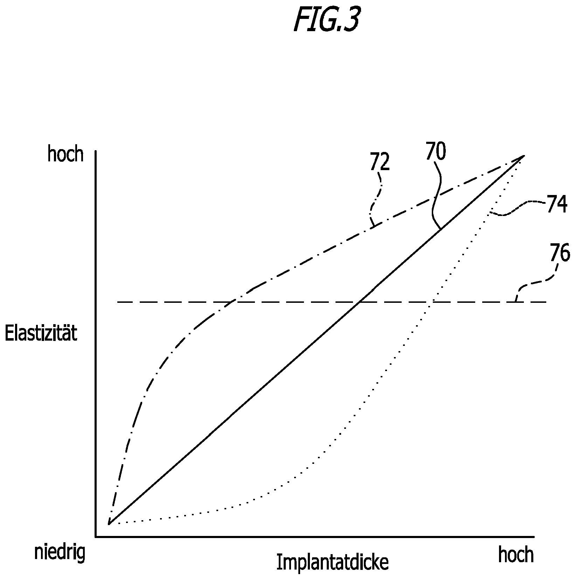

In Figur 3 sind insgesamt drei verschiedene, stetige Elastizitätsverläufe 70, 72 und 74 beispielhaft dargestellt, die mit dem beschriebenen Verfahren zur Ausbildung von Gelenkimplantatteilen erreicht werden können. Der Elastizitätsverlauf 70 zeigt schematisch eine lineare Abhängigkeit der Elastizität von einer Implantatdicke. Der Elastizitätsverlauf 72 zeigt eine höhere Elastizität bei vergleichbarer Implantatdicke als der Elastizitätsverlauf 70. Der Elastizitätsverlauf 74 zeigt schematisch eine niedrigere Elastizität im Vergleich zum Elastizitätsverlauf 70 bei zunehmender Implantatdicke.

Im Vergleich zu den möglichen Elastizitätsverläufen 70, 72 und 74 ist in Figur 3 ein Elastizitätsverlauf 76 dargestellt, welchen man bei einem gleichmäßigen Gefügeaufbau erhält, wie er bei am Markt befindlichen Implantaten üblich ist. Unabhängig von einer Implantatdicke ist dabei eine Elastizität des Gelenkimplantatteils im Wesentlichen konstant.

Mit dem oben beschriebenen erfindungsgemäßen Verfahren ist es insbesondere möglich, Bauteiloptimierungen zu realisieren, welche die Ausbildung von beispielsweise Gelenkimplantatteilen 14 mit einer reduzierten Masse ermöglichen. Diese sorgen für ein deutlich besseres Wohlbefinden des Patienten. Zudem sind Bauteiloptimierungen in allen Dimensionen möglich. Es ist insbesondere möglich, sowohl eine Elastizität im Inneren des Gelenkimplantatteils zu beeinflussen als auch eine Elastizität in Abhängigkeit einer Implantatdicke zu variieren. Ferner kann auch eine Elastizität des Gelenkimplantatteils im Bereich eines Knochenkontakts an die Elastizität eines menschlichen oder tierischen Knochens angepasst werden.

Anwendbar sind die beschriebenen Verfahren grundsätzlich bei allen Arten von Gelenkimplantatteilen, also insbesondere zur Ausbildung von Gelenkimplantatteilen von Hüft- und Kniegelenkendoprothesen aller Versorgungsbereiche, also sowohl bei der Erstversorgung und bei der Revision als auch bei der Tumorversorgung eines Patienten.

Das vorgeschlagene Lasersintern zur Ausbildung der Gelenkimplantatteile ermöglicht es insbesondere, alles Optionen, die dieses Verfahren bereitstellt, auszunutzen. Durch den additiven Aufbau von Gelenkimplantatteilen ist die Gestaltung von Strukturen, Gefügen und Materialzusammensetzungen möglich, welche mit bisherigen, gießtechnischen oder spanenden Bearbeitungsverfahren nicht denkbar sind.

Mit den vorgeschlagenen Verfahren ist es möglich, Implantate auszubilden, bei denen sowohl eine Optimierung einer Bauteilgeometrie in Abhängigkeit eines Gewichts und zusätzlich auch eine Elastizitätsänderung in Abhängigkeit einer Materialdicke Berücksichtigung finden kann.

In der beschriebenen Weise lassen sich vielfältige Arten von Gelenkimplantatteilen ausbilden und Ärzten zur Implantation bereitstellen, welche eine dauerhafte Stabilität sowie ein deutlich verbessertes Zusammenwirken mit verbliebenem Knochen des Patienten ermöglichen.

### Bezugszeichenliste

- 10: xy-Ebene
- 12: yz-Ebene
- 14: Gelenkimplantatteil
- 16: Gelenkendoprothese
- 18: Gelenkimplantatteil
- 20: Gelenkimplantatteil
- 22: Kniegelenkendoprothese
- 24: Femurteil
- 26: Tibiateil
- 28: Schaft
- 30: Meniskusteil
- 32: Knochenanlagefläche
- 34: Gelenkfläche
- 36: Gelenkfläche
- 38: Knochenanlageflächenbereich
- 40: innerer Bereich
- 42: Knochenanlagefläche
- 44: Knochenfläche
- 46: Knochen
- 48: Hohlraum
- 50: Hohlraum
- 52: erster Volumenbereich
- 54: Knochenanlageflächenbereich
- 56: Knochenanlagefläche
- 58: zweiter Volumenbereich
- 60: Gelenkflächenbereich
- 62: Gelenkfläche
- 64: dritter Volumenbereich
- 66: Hohlraum
- 68: Oberfläche
- 70: Elastizitätsverlauf
- 72: Elastizitätsverlauf
- 74: Elastizitätsverlauf
- 76: Elastizitätsverlauf

## Patentansprüche

1. Gelenkimplantatteil (14, 18, 20) einer Gelenkendoprothese (16), welches Gelenkimplantatteil (14, 18, 20) mindestens einen ersten Volumenbereich (52), mindestens einen zweiten Volumenbereich (58) und mindestens einen dritten Volumenbereich (64) umfasst, welcher mindestens eine erste Volumenbereich (52) einen Knochenanlageflächenbereich (38; 54) mit mindestens einer Knochenanlagefläche (32; 56) definiert, welcher mindestens eine zweite Volumenbereich (58) einen Gelenkflächenbereich (60) mit mindestens einer Gelenkfläche (36; 62) definiert und welcher mindestens eine dritte Volumenbereich (64) weder eine Knochenanlagefläche noch eine Gelenkfläche umfasst, wobei sich ein Elastizitätsmodul im mindestens einen ersten und/oder im mindestens einen zweiten und/oder im mindestens einen dritten Volumenbereich (52, 58, 64) in mindestens einer Raumrichtung stetig oder im Wesentlichen stetig oder unstetig ändert und wobei sich eine Festigkeit im mindestens einen ersten und/oder im mindestens einen zweiten und/oder im mindestens einen dritten Volumenbereich (52, 58, 64) in mindestens einer Raumrichtung stetig oder im Wesentlichen stetig oder unstetig ändert, wobei der mindestens eine erste Volumenbereich (52) und der mindestens eine zweite Volumenbereich (58) und der mindestens eine dritte Volumenbereich (64) mindestens einen Hohlraum (66) aufweisen, wobei eine Mehrzahl von Hohlräumen (66) ausgebildet ist, wobei ein durch die Mehrzahl von Hohlräumen (66) definiertes Gesamtvolumen im mindestens einen dritten Volumenbereich (64) größer ist als im mindestens einen ersten und im mindestens einen zweiten Volumenbereich (52, 58) und wobei das Gelenkimplantatteil (14) in Form eines an einem Femur festlegbaren Femurteils (24) einer Kniegelenkendoprothese (22) und einstückig ausgebildet ist.

2. Gelenkimplantatteil nach Anspruch 1, **dadurch gekennzeichnet, dass** der mindestens eine erste Volumenbereich (52) eine Elastizität mit einem Elastizitätsmodul aufweist, insbesondere im Bereich der mindestens einen Knochenanlagefläche (32; 56), welcher einem Elastizitätsmodul eines menschlichen oder tierischen Knochens (46) entspricht oder im Wesentlichen entspricht.

3. Gelenkimplantatteil nach einem der voranstehenden Ansprüche, **dadurch gekennzeichnet, dass**
a) ein Elastizitätsmodul des mindestens einen ersten und/oder des mindestens einen zweiten und/oder des mindestens einen dritten Volumenbereichs (52, 58, 64) in einem Bereich von etwa 0,2 kN/mm² bis etwa 100 kN/mm² liegt
und/oder
b) die Festigkeit des mindestens einen ersten und/oder des mindestens einen zweiten und/oder des mindestens einen dritten Volumenbereichs (52, 58, 64) in einem Bereich von etwa 0,2 kN/mm² bis etwa 100 kN/mm² liegt
und/oder
c) der mindestens eine erste Volumenbereich (52) eine innere Struktur aufweist, welche im Wesentlichen einem inneren Aufbau eines menschlichen oder tierischen Knochens (46) entspricht.

4. Gelenkimplantatteil nach einem der voranstehenden Ansprüche, **dadurch gekennzeichnet, dass** es durch Sintern, insbesondere Lasersintern, aus mindestens einem sinterfähigen Material hergestellt ist,
wobei das Gelenkimplantatteil insbesondere aus mindestens zwei, vor dem Sintern gemischte Materialien hergestellt ist.

5. Gelenkimplantatteil nach Anspruch 4, **dadurch gekennzeichnet, dass** das mindestens eine sinterfähige Material ein Metall und/oder ein Kunststoff ist.

6. Gelenkimplantatteil nach einem der voranstehenden Ansprüche, **dadurch gekennzeichnet, dass** der mindestens eine erste und/oder der mindestens eine zweite und/oder der mindestens eine dritte Volumenbereich (52, 58, 64) aus unterschiedlichen Materialien ausgebildet sind,
wobei insbesondere sich ein Anteil unterschiedlicher Materialen beim Übergang zwischen zwei der drei unterschiedlichen Volumenbereiche (52, 58, 64) in mindestens einer Raumrichtung stetig oder im Wesentlichen stetig ändert oder unstetig ändert.

7. Gelenkimplantatteil nach einem der voranstehenden Ansprüche, **dadurch gekennzeichnet, dass**
a) der mindestens eine Hohlraum (66) in Form eines abgeschlossenen, vollständig umschlossenen Hohlraums (66) ausgebildet ist und/oder
b) mindestens ein Teil der Hohlräume (66) miteinander in Fluidverbindung stehen

8. Gelenkimplantatteil nach einem der voranstehenden Ansprüche, **dadurch gekennzeichnet, dass**
a) der mindestens eine Hohlraum (66) in Form einer Vertiefung der Knochenanlagefläche (32; 56) ausgebildet ist
und/oder
b) die mindestens eine Gelenkfläche (36; 62) in Form einer geschlossenen Materialschicht und/oder glatt und/oder poliert ausgebildet ist.

9. Gelenkendoprothese (16) umfassend mindestens ein Gelenkimplantatteil (14, 18, 20), **dadurch gekennzeichnet, dass** das mindestens eine Gelenkimplantatteil (14, 18, 20) in Form eines Gelenkimplantatteils (14, 18, 20) nach einem der voranstehenden Ansprüche ausgebildet ist,
wobei die Gelenkendoprothese insbesondere in Form einer Kniegelenkendoprothese (22) ausgebildet ist.

10. Verfahren zum Herstellen eines Gelenkimplantatteils (14, 18, 20) einer Gelenkendoprothese (16), welches Gelenkimplantatteil (14, 18, 20) mit mindestens einem ersten Volumenbereich (52), mindestens einem zweiten Volumenbereich (58) und mindestens einem dritten Volumenbereich (64) ausgebildet wird, welcher mindestens eine erste Volumenbereich (52) in Form eines Knochenanlageflächenbereichs (38; 54) mit mindestens einer Knochenanlagefläche (32; 56) ausgebildet wird, welcher mindestens eine zweite Volumenbereich (58) in Form eines Gelenkflächenbereichs (60) mit mindestens einer Gelenkfläche (36; 62) ausgebildet wird und welcher mindestens eine dritte Volumenbereich (64) weder mit einer Knochenanlagefläche noch mit einer Gelenkfläche ausgebildet wird, wobei der mindestens eine erste und/oder der mindestens eine zweite und/oder der mindestens eine dritte Volumenbereich (52, 58, 64) mit einem Elastizitätsmodul ausgebildet werden, welcher sich in mindestens einer Raumrichtung stetig oder im Wesentlichen stetig oder unstetig ändert, und wobei der mindestens eine erste und/oder der mindestens eine zweite und/oder der mindestens eine dritte Volumenbereich (52, 58, 64) mit einer Festigkeit ausgebildet werden, welche sich in mindestens einer Raumrichtung stetig oder im Wesentlichen stetig oder unstetig ändert, wobei der mindestens eine erste Volumenbereich (52) und der mindestens eine zweite Volumenbereich (58) und der mindestens eine dritte Volumenbereich (64) mit mindestens einem Hohlraum (66) ausgebildet werden, wobei eine Mehrzahl von Hohlräumen (66) ausgebildet wird, wobei die Mehrzahl von Hohlräumen (66) derart ausgebildet wird, dass ein durch die Mehrzahl von Hohlräumen (66) definiertes Gesamtvolumen im mindestens einen dritten Volumenbereich (64) größer ist als im mindestens einen ersten und/oder im mindestens einen zweiten Volumenbereich (52, 58) und wobei das Gelenkimplantatteil (14, 18, 20) in Form eines an einem Femur festlegbaren Femurteils (24) einer Kniegelenkendoprothese (22) und einstückig ausgebildet wird.

11. Verfahren nach Anspruch 10, **dadurch gekennzeichnet, dass** der mindestens eine erste Volumenbereich (52) mit einer Elastizität mit einem Elastizitätsmodul ausgebildet wird, insbesondere im Bereich der mindestens einen Knochenanlagefläche (32; 56), welcher einem Elastizitätsmodul eines menschlichen oder tierischen Knochens (46) entspricht.

12. Verfahren nach Anspruch 10 oder 11, **dadurch gekennzeichnet, dass** das Gelenkimplantatteil (14, 18, 20) durch Sintern, insbesondere Lasersintern, aus mindestens einem sinterfähigen Material hergestellt wird.

13. Verfahren nach Anspruch 12, **dadurch gekennzeichnet, dass**
a) das Gelenkimplantatteil (14, 18, 20) aus mindestens zwei Materialien hergestellt wird, welche vor dem Sintern gemischt werden, und/oder
b) als das mindestens eine sinterfähige Material mindestens ein Metall und/oder mindestens ein Kunststoff eingesetzt werden.

14. Verfahren nach einem der Ansprüche 10 bis 13, **dadurch gekennzeichnet, dass**
a) der mindestens eine Hohlraum (66) in Form eines abgeschlossenen, vollständig umschlossenen Hohlraums (66) ausgebildet wird und/oder
b) eine Mehrzahl von Hohlräumen (66) ausgebildet wird derart, dass mindestens ein Teil der Hohlräume (66) miteinander in Fluidverbindung steht
und/oder
c) der mindestens eine Hohlraum (66) in Form einer Vertiefung der Knochenanlagefläche (32; 56) ausgebildet wird
und/oder
d) die mindestens eine Gelenkfläche (36; 62) in Form einer geschlossenen Materialschicht und/oder glatt und/oder poliert ausgebildet wird.

15. Verfahren nach Anspruch 14, **dadurch gekennzeichnet, dass**
a) der mindestens eine Hohlraum (66) in Form eines abgeschlossenen, vollständig umschlossenen Hohlraums (66) ausgebildet wird und/oder
b) eine Mehrzahl von Hohlräumen (66) ausgebildet wird derart, dass mindestens ein Teil der Hohlräume (66) miteinander in Fluidverbindung steht,
und/oder
c) der mindestens eine Hohlraum (66) in Form einer Vertiefung der Knochenanlagefläche (32; 56) ausgebildet wird.

## Claims

1. Joint implant part (14, 18, 20) of a joint endoprosthesis (16), which joint implant part (14, 18, 20) comprises at least one first volume region (52), at least one second volume region (58) and at least one third volume region (64), which at least one first volume region (52) defines a bone contact surface region (38; 54) with at least one bone contact surface (32; 56), which at least one second volume region (58) defines a joint surface region (60) with at least one joint surface (36; 62) and which at least one third volume region (64) comprises neither a bone contact surface nor a joint surface, wherein a modulus of elasticity in the at least one first and/or in the at least one second and/or in the at least one third volume region (52, 58, 64) changes continuously or substantially continuously or discontinuously in at least one spatial direction and wherein a firmness in the at least one first and/or in the at least one second and/or in the at least one third volume region (52, 58, 64) changes continuously or substantially continuously or discontinuously in at least one spatial direction, wherein the at least one first volume region (52) and the at least one second volume region (58) and the at least one third volume region (64) are formed with at least one cavity (66), wherein a plurality of cavities (66) are formed, wherein a total volume defined by the plurality of cavities (66) in the at least one third volume region (64) is greater than in the at least one first and in the at least one second volume region (52, 58) and wherein the joint implant part (14) is in the form of a femur part (24) of a knee joint endoprosthesis (22), which is fixable to a femur, and is of one-piece formation.

2. Joint implant part in accordance with claim 1, **characterized in that** the at least one first volume region (52) has an elasticity with a modulus of elasticity, in particular, in the region of the at least one bone contact surface (32; 56), which corresponds or corresponds substantially to a modulus of elasticity of a bone (46) of a human being or an animal.

3. Joint implant part in accordance with any one of the preceding claims, **characterized in that**
a) a modulus of elasticity of the at least one first and/or of the at least one second and/or of the at least one third volume region (52, 58, 64) lies in a range of from approximately 0.2 kN/mm² to approximately 100 kN/mm²
and/or
b) the firmness of the at least one first and/or of the at least one second and/or of the at least one third volume region (52, 58, 64) lies in a range of from approximately 0.2 kN/mm² to approximately 100 kN/mm²
and/or
c) the at least one first volume region (52) has an inner structure which corresponds substantially to an inner construction of a bone (46) of a human being or an animal.

4. Joint implant part in accordance with any one of the preceding claims, **characterized in that** it is produced by sintering, in particular, laser sintering, from at least one sinterable material,
wherein, in particular, the joint implant part is produced from at least two materials mixed before the sintering.

5. Joint implant part in accordance with claim 4, **characterized in that** the at least one sinterable material is a metal and/or a plastic material.

6. Joint implant part in accordance with any one of the preceding claims, **characterized in that** the at least one first and/or the at least one second and/or the at least one third volume region (52, 58, 64) are formed from different materials,
wherein, in particular, a proportion of different materials in the transition between two of the three different volume regions (52, 58, 64) changes continuously or substantially continuously or changes discontinuously in at least one spatial direction.

7. Joint implant part in accordance with any one of the preceding claims, **characterized in that**
a) the at least one cavity (66) is in the form of a closed, completely enclosed cavity (66)
and/or
b) at least some of the cavities (66) are in fluid communication with one another.

8. Joint implant part in accordance with any one of the preceding claims, **characterized in that**
a) the at least one cavity (66) is in the form of a depression of the bone contact surface (32; 56)
and/or
b) the at least one joint surface (36; 62) is in the form of a closed layer of material and/or is smooth and/or is polished.

9. Joint endoprosthesis (16) comprising at least one joint implant part (14, 18, 20), **characterized in that** the at least one joint implant part (14, 18, 20) is in the form of a joint implant part (14, 18, 20) in accordance with any one of the preceding claims,
wherein, in particular, the joint endoprosthesis is in the form of a knee joint endoprosthesis (22).

10. Method for producing a joint implant part (14, 18, 20) of a joint endoprosthesis (16), which joint implant part (14, 18, 20) is formed with at least one first volume region (52), at least one second volume region (58) and at least one third volume region (64), which at least one first volume region (52) is made in the form of a bone contact surface region (38; 54) with at least one bone contact surface (32; 56), which at least one second volume region (58) is made in the form of a joint surface region (60) with at least one joint surface (36; 62), and which at least one third volume region (64) is formed neither with a bone contact surface nor with a joint surface, wherein the at least one first and/or the at least one second and/or the at least one third volume region (52, 58, 64) are formed with a modulus of elasticity which changes continuously or substantially continuously or discontinuously in at least one spatial direction, and wherein the at least one first and/or the at least one second and/or the at least one third volume region (52, 58, 64) are formed with a firmness which changes continuously or substantially continuously or discontinuously in at least one spatial direction, wherein the at least one first volume region (52) and the at least one second volume region (58) and the at least one third volume region (64) are formed with at least one cavity (66), wherein a plurality of cavities (66) are formed, wherein the plurality of cavities (66) are formed such that a total volume defined by the plurality of cavities (66) in the at least one third volume region (64) is greater than in the at least one first and/or in the at least one second volume region (52, 58) and wherein the joint implant part (14, 18, 20) is in the form of a femur part (24) of a knee joint endoprosthesis (22), which is fixable to a femur, and is of one-piece formation.

11. Method in accordance with claim 10, **characterized in that** the at least one first volume region (52) is formed with an elasticity with a modulus of elasticity, in particular, in the region of the at least one bone contact surface (32; 56), which corresponds to a modulus of elasticity of a bone (46) of a human being or an animal.

12. Method in accordance with claim 10 or 11, **characterized in that** the joint implant part (14, 18, 20) is produced by sintering, in particular, laser sintering, from at least one sinterable material.

13. Method in accordance with claim 12, **characterized in that**
a) the joint implant part (14, 18, 20) is produced from at least two materials which are mixed before the sintering,
and/or
b) at least one metal and/or at least one plastic material are used as the at least one sinterable material.

14. Method in accordance with any one of claims 10 to 13, **characterized in that**
a) the at least one cavity (66) is made in the form of a closed, completely enclosed cavity (66)
and/or
b) a plurality of cavities (66) are formed such that at least some of the cavities (66) are in fluid communication with one another,
and/or
c) the at least one cavity (66) is made in the form of a depression of the bone contact surface (32; 56)
and/or
d) the at least one joint surface (36; 62) is made in the form of a closed layer of material and/or is formed so as to be smooth and/or is formed so as to be polished.

15. Method in accordance with claim 14, **characterized in that**
a) the at least one cavity (66) is made in the form of a closed, completely enclosed cavity (66)
and/or
b) a plurality of cavities (66) are formed such that at least some of the cavities (66) are in fluid communication with one another,
and/or
c) the at least one cavity (66) is made in the form of a depression of the bone contact surface (32; 56).

## Revendications

1. Partie d'implant d'articulation (14, 18, 20) d'une endoprothèse d'articulation (16), laquelle partie d'implant d'articulation (14, 18, 20) comprend au moins une première zone de volume (52), au moins une seconde zone de volume (58) et au moins une troisième zone de volume (64), laquelle au moins une première zone de volume (52) définit une zone de surface de contact avec l'os (38; 54) avec au moins une surface de contact avec l'os (32; 56), laquelle au moins une seconde zone de volume (58) définit une zone de surface d'articulation (60) avec au moins une surface d'articulation (36; 62) et laquelle au moins une troisième zone de volume (64) ne comprend ni une surface de contact avec l'os ni une surface d'articulation, où un module d'élasticité dans au moins une première et/ou dans au moins une seconde et/ou dans au moins une troisième zone de volume (52, 58, 64) change de manière continue ou sensiblement continue ou discontinue dans au moins une direction spatiale et où une résistance dans au moins une première et/ou dans au moins une seconde et/ou dans au moins une troisième zone de volume (52, 58, 64) change de manière continue ou sensiblement continue ou discontinue dans au moins une direction spatiale, où la au moins une première zone de volume (52) et la au moins une seconde zone de volume (58) et la au moins une troisième zone de volume (64) présentent au moins une cavité (66), où une pluralité de cavités (66) est formée, où un volume total défini par la pluralité de cavités (66) est plus grand dans au moins une troisième zone de volume (64) que dans au moins une première et dans au moins une seconde zone de volume (52, 58) et où la partie d'implant d'articulation (14) est formée sous forme d'une partie fémur (24) qui peut être fixée à un fémur d'une endoprothèse d'articulation du genou (22) et est formée d'une seule pièce.

2. Partie d'implant d'articulation selon la revendication 1, **caractérisée en ce que** la au moins une première zone de volume (52) présente une élasticité avec un module d'élasticité, en particulier dans le domaine de la au moins une surface de contact avec l'os (32; 56), qui correspond ou correspond sensiblement à un module d'élasticité d'un os humain ou animal (46).

3. Partie d'implant d'articulation selon l'une des revendications précédentes, **caractérisée en ce que**
a) un module d'élasticité de la au moins une première et/ou de la au moins une seconde et/ou de la au moins une troisième zone de volume (52, 58, 64) est situé dans un domaine d'environ 0,2 kN/mm² à environ 100 kN/mm²
et/ou
b) la résistance de la au moins une première et/ou de la au moins une seconde et/ou de la au moins une troisième zone de volume (52, 58, 64) est située dans un domaine d'environ 0,2 kN/mm² à environ 100 kN/mm²
et/ou
c) la au moins une première zone de volume (52) présente une structure interne qui correspond sensiblement à une constitution interne d'un os humain ou animal (46).

4. Partie d'implant d'articulation selon l'une des revendications précédentes, **caractérisée en ce qu'**elle est produite par frittage, en particulier frittage laser, à partir d'au moins un matériau pouvant être fritté,
où la partie d'implant d'articulation est produite en particulier à partir d'au moins deux matériaux mélangés avant le frittage.

5. Partie d'implant d'articulation selon la revendication 4, **caractérisée en ce que** le au moins un matériau pouvant être fritté est un métal et/ou une matière plastique.

6. Partie d'implant d'articulation selon l'une des revendications précédentes, **caractérisée en ce que** la au moins une première et/ou la au moins une seconde et/ou la au moins une troisième zone de volume (52, 58, 64) sont formées à partir de matériaux différents,
où en particulier une proportion de matériaux différents change de manière continue ou sensiblement continue ou change de manière discontinue dans au moins une direction spatiale lors de la transition entre deux des trois zones de volume différentes (52, 58, 64).

7. Partie d'implant d'articulation selon l'une des revendications précédentes, **caractérisée en ce que**
a) la au moins une cavité (66) est formée sous forme d'une cavité fermée, complètement entourée (66) et/ou
b) au moins une partie des cavités (66) sont en liaison fluidique les unes avec les autres.

8. Partie d'implant d'articulation selon l'une des revendications précédentes, **caractérisée en ce que**
a) la au moins une cavité (66) est formée sous forme d'un évidement de la surface de contact avec l'os (32; 56) et/ou
b) la au moins une surface d'articulation (36; 62) est formée sous forme d'un couche de matériau fermée et/ou est formée lisse et/ou polie.

9. Endoprothèse d'articulation (16) comprenant au moins une partie d'implant d'articulation (14, 18, 20), **caractérisée en ce que** la au moins une partie d'implant d'articulation (14, 18, 20) est formée sous forme d'une partie d'implant d'articulation (14, 18, 20) selon l'une des revendications précédentes, où l'endoprothèse d'articulation est formée en particulier sous forme d'une endoprothèse d'articulation du genou (22).

10. Procédé de production d'une partie d'implant d'articulation (14, 18, 20) d'une endoprothèse d'articulation (16), laquelle partie d'implant d'articulation (14, 18, 20) est formée avec au moins une première zone de volume (52), au moins une seconde zone de volume (58) et au moins une troisième zone de volume (64), laquelle au moins une première zone de volume (52) est formée sous forme d'une zone de surface de contact avec l'os (38; 54) avec au moins une surface de contact avec l'os (32; 56), laquelle au moins une seconde zone de volume (58) est formée sous forme d'une zone de surface d'articulation (60) avec au moins une surface d'articulation (36; 62) et laquelle au moins une troisième zone de volume (64) n'est formée ni avec une surface de contact avec l'os ni avec une surface d'articulation, où la au moins une première et/ou la au moins une seconde et/ou la au moins une troisième zone de volume (52, 58, 64) sont formées avec un module d'élasticité qui change de manière continue ou de manière sensiblement continue ou discontinue dans au moins une direction spatiale, et où la au moins une première et/ou la au moins une seconde et/ou la au moins une troisième zone de volume (52, 58, 64) sont formées avec une résistance qui change de manière continue ou de manière sensiblement continue ou discontinue dans au moins une direction spatiale, où la au moins une première zone de volume (52) et la au moins une seconde zone de volume (58) et la au moins une troisième zone de volume (64) sont formées avec au moins une cavité (66), où une pluralité de cavités (66) sont formées, où la pluralité de cavités (66) est formée de telle sorte qu'un volume total défini par la pluralité de cavités (66) est plus grand dans au moins une troisième zone de volume (64) que dans au moins une première et/ou dans au moins une seconde zone de volume (52, 58) et où la partie d'implant d'articulation (14, 18, 20) est formée sous forme d'une partie fémur (24) d'une endoprothèse d'articulation du genou (22) qui peut être fixée à un fémur et est formée d'une seule pièce.

11. Procédé selon la revendication 10, **caractérisé en ce que** la au moins une première zone de volume (52) est formée avec une élasticité avec un module d'élasticité, en particulier dans le domaine de la au moins une surface de contact avec l'os (32; 56), qui correspond à un module d'élasticité d'un os humain ou animal (46).

12. Procédé selon la revendication 10 ou 11, **caractérisé en ce que** la partie d'implant d'articulation (14, 18, 20) est produite par frittage, en particulier frittage laser, à partir d'au moins un matériau pouvant être fritté.

13. Procédé selon la revendication 12, **caractérisé en ce que**
a) la partie d'implant d'articulation (14, 18, 20) est produite à partir d'au moins deux matériaux qui sont mélangés avant le frittage, et/ou
b) au moins un métal et/ou au moins une matière plastique sont utilisés comme le au moins un matériau pouvant être fritté.

14. Procédé selon l'une quelconque des revendications 10 à 13, **caractérisé en ce que**
a) la au moins une cavité (66) est formée sous forme d'une cavité fermée, totalement entourée (66)
et/ou
b) une pluralité de cavités (66) est formée de telle sorte qu'au moins une partie des cavités (66) sont en communication fluidique les unes avec les autres
et/ou
c) la au moins une cavité (66) est formée sous forme d'un évidement dans la surface de contact avec l'os (32; 56)
et/ou
d) la au moins une surface d'articulation (36; 62) est formée sous forme d'une couche de matériau fermée et/ou est formée lisse et/ou polie.

15. Procédé selon la revendication 14, **caractérisé en ce que**
a) la au moins une cavité (66) est formée sous forme d'une cavité fermée, complètement entourée (66)
et/ou
b) une pluralité de cavités (66) est formée de telle manière qu'au moins une partie des cavités (66) sont en communication fluidique les unes avec les autres,
et/ou
c) la au moins une cavité (66) est formée sous forme d'un évidement dans la surface de contact avec l'os (32; 56).
